# EUROPEAN PATENT APPLICATION

(11) **EP 1 510 521 A1**
(43) Date of publication of application: **02.03.2005**
(21) Application number: 04019610.7
(22) Date of filing: 17.12.2001
(51) Int. Cl.: C07H 15/26, A61K 31/70

(54) **Sugar-substituted 2-Azetidinones useful as hypocholesterolemic agents**

(30) Priority: 20.12.2000 US 256875 P
(62) Divisional of application: 01991315.1
(71) Applicant: Schering Corporation, Kenilworth, NJ 07033-0530 (US)
(72) Inventor: Ghosal, Anima, Edison, NJ 08817 (US); Zbaida, Shmuel, East Brunswick, NJ 08816 (US); Chowdhury, Swapan K., Warren, NJ 07059 (US); Iannucci, Robert M., Hampton, NJ 08827 (US); Feng, Wenqing, Chatham, NJ 07928 (US); Alton, Kevin B., Cedar Knolls, NJ 07927 (US); Patrick, James E., Belle Mead, NJ 08502 (US); Davis, Harry R., Berkeley Heights NJ 07922 (US)
(74) Representative: HOFFMANN - EITLE

(57) **Abstract**

Hypocholesterolemic sugar-substituted 2-azetidinone compounds of the formula: are disclosed, as well as a method of lowering cholesterol by administering said compounds, pharmaceutical compositions containing them, and the combination of a sugar-substituted 2-azetidinone cholesterol-lowering agent and a cholesterol biosynthesis inhibitor for the treatment and prevention of atherosclerosis.

## Description

### BACKGROUND OF THE INVENTION

The present invention relates to sugar-substituted 2-azetidinones useful as hypocholesterolemic agents in the treatment and prevention of atherosclerosis, and to a combination of a sugar-substituted 2-azetidinone of this invention and a cholesterol biosynthesis inhibitor for the treatment and prevention of atherosclerosis.

Atherosclerotic coronary heart disease represents the major cause for death and cardiovascular morbidity in the western world. Risk factors for atherosclerotic coronary heart disease include hypertension, diabetes mellitus, family history, male gender, cigarette smoke and serum cholesterol. A total cholesterol level in excess of 225-250 mg/dl is associated with significant elevation of risk.

Cholesteryl esters are a major component of atherosclerotic lesions and the major storage form of cholesterol in arterial wall cells. Formation of cholesteryl esters is also a key step in the intestinal absorption of dietary cholesterol. In addition to regulation of dietary cholesterol, the regulation of whole-body cholesterol homeostasis in humans and animals involves modulation of cholesterol biosynthtesis, bile acid biosynthesis, and the catabolism of the cholesterol-containing plasma lipoproteins. The liver is the major organ responsible for cholesterol biosynthesis and catabolism and, for this reason, it is a prime determinant of plasma cholesterol levels. The liver is the site of synthesis and secretion of very low density lipoproteins (VLDL) which are subsequently metabolized to low density lipoproteins (LDL) in the circulation. LDL are the predominant cholesterol-carrying lipoproteins in the plasma and an increase in their concentration is correlated with increased atherosclerosis.

When cholesterol absorption in the intestines is reduced, by whatever means, less cholesterol is delivered to the liver. The consequence of this action is a decreased hepatic lipoprotein (VLDL) production and an increase in the hepatic clearance of plasma cholesterol, mostly as LDL. Thus, the net effect of an inhibition of intestinal cholesterol absorption is a decrease in plasma cholesterol levels.

Several 2-azetidinone compounds have been reported as being useful in lowering cholesterol and/or in inhibiting the formation of cholesterol-containing lesions in mammalian arterial walls: WO 93/02048 describes 2-azetidinone compounds wherein the 3-position substituent is arylalkylene, arylalkenylene or arylalkylene wherein the alkylene, alkenylene or alkyleneportion is interrupted by a hetero atom, phenylene or cycloalkylene; WO 94/17038 describes 2-azetidinone compounds wherein the 3-position substituent is an arylalkylspirocyclic group; WO 95/08532 describes 2-azetidinone compounds wherein the 3-position substituent is an arylalkylene group substituted in the alkylene portion by a hydroxy group; PCT/US95/03196 describes compounds wherein the 3-position substituent is an aryl(oxo or thio)alkylene group substituted in the alkylene portion by a hydroxy group; and U.S. Serial No. 08/463,619, filed June 5, 1995, describes the preparation of compounds wherein the 3-position substituent is an arylalkylene group substituted in the alkylene portion by a hydroxy group, and wherein the alkylene group is attached to the azetidinone ring by a -S(O)₀₋₂- group.

Also, European Patent 199,630B1 and European Patent Application 337,549A1 disclose elastase inhibitory substituted azetidinones useful in treating inflammatory conditions resulting in tissue destruction which are associated with various disease states, e.g., atherosclerosis.

Other known hypocholesterolemics include plant extracts such as sapogenins, in particular tigogenin and diosgenin. Glycoside derivatives of tigogenin and/or diosgenin are disclosed in WO 94/00480 and WO 95/18143.

The inhibition of cholesterol biosynthesis by 3-hydroxy-3-methyl-glutaryl coenzyme A reductase (EC1.1.1.34) inhibitors has been shown to be an effective way to reduce plasma cholesterol (Witzum, *Circulation, 80,* 5 (1989), p. 1101-1114) and reduce atherosclerosis. Combination therapy of an HMG CoA reductase inhibitor and a bile acid sequestrant has been demonstrated to be more effective in human hyperlipidemic patients than either agent in monotherapy (Illingworth, *Drugs, 36* (Suppl. 3) (1988), p. 63-71).

### SUMMARY OF THE INVENTION

The present invention relates to sugar-substituted 2-azetidinones, especially to glucose-derived conjugates of cholesterol-lowering 2-azetidinones having an aryl or substituted aryl group as a substituent at the 1-position and having a hydroxy-substituted phenyl group, especially a 4-hydroxyphenyl group, at the 4-position. Examples of sugars useful as substituents in the present invention include but are not limited to hexose, and ribose.

Compounds of the present invention are represented by the formula I: 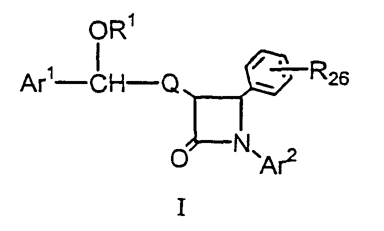 or a pharmaceutically acceptable salt thereof, wherein
R²⁶ is selected from the group consisting of:
   a) OCH₃;
   b) fluorine and
   c) chlorine.
R¹ is selected from the group consisting of H, 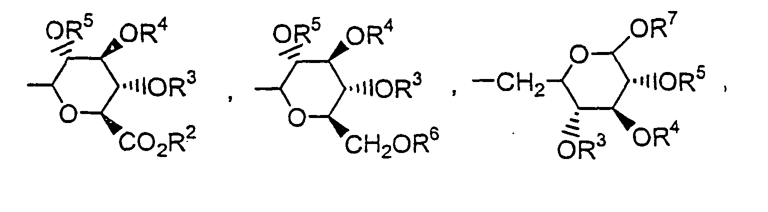 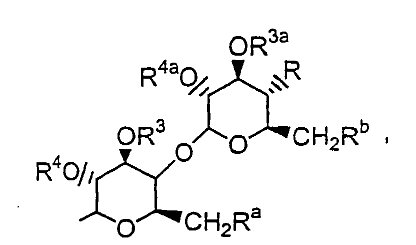 -SO₃H; natural and unnatural amino acids.
R, R^{a} and R^{b} are independently selected from the group consisting of H, - OH, halogeno, -NH₂, azido, (C₁-C₆)alkoxy(C₁-C₆)-alkoxy and -W-R³⁰;
W is independently selected from the group consisting of - NH-C(O)-, -O-C(O)-, -O-C(O)-N(R³¹)-, -NH-C(O)-N(R³¹)- and -O-C(S)-N(R³¹)-;
R² and R⁶ are independently selected from the group consisting of H, (C₁-C₆)alkyl, aryl and aryl(C₁-C₆)alkyl;
R³, R⁴, R⁵, R⁷, R^{3a} and R^{4a} are independently selected from the group consisting of H, (C₁-C₆)alkyl, aryl(C₁-C₆)alkyl, -C(O)(C₁-C₆)alkyl and -C(O)aryl;
R³⁰ is independently selected form the group consisting of R³²-substituted T, R³²-substituted-T-(C₁-C₆)alkyl, R³²-substituted-(C₂-C₄)alkenyl, R³²-substituted-(C₁-C₆)alkyl, R³²-substituted-(C₃-C7)cycloalkyl and R³²-substituted-(C₃-C7)cycloalkyl(C₁-C₆)alkyl;
R³¹ is independently selected from the group consisting of H and (C₁-C₄)alkyl;
T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
R³² is independently selected from 1-3 substituents independently selected from the group consisting of H, halogeno, (C₁-C₄)alkyl, -OH, phenoxy, -CF₃, -NO₂, (C₁-C₄)alkoxy, methylenedioxy, oxo, (C₁-C₄)alkylsulfanyl, (C₁-C₄)alkylsulfinyl, (C₁-C₄)alkylsulfonyl, -N(CH₃)₂, -C(O)-NH(C₁-C₄)alkyl, -C(O)-N((C₁-C₄)alkyl)₂, -C(O)-(C₁-C₄)alkyl, -C(O)-(C₁-C₄)alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a (C₁-C₄)alkoxycarbonyl-substituted pyrrolidinyl, piperidinyl, N-methylpiperazinyl, indolinyl or morpholinyl group;
Ar¹ is aryl or R¹⁰-substituted aryl;
Ar² is aryl or R¹¹ -substituted aryl;
Q is -(CH₂)_{q}-, wherein q is 2-6, or, with the 3-position ring carbon of the azetidinone, forms the spiro group 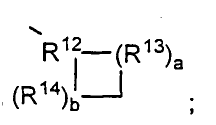
R¹² is 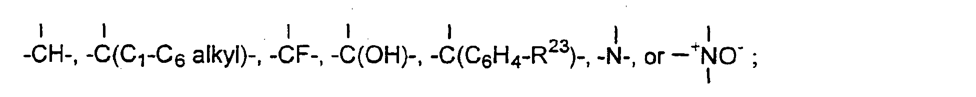
R¹³ and R¹⁴ are independently selected from the group consisting of -CH₂-, -CH(C₁-C₆ alkyl)-, -C(di-(C₁-C₆) alkyl), -CH=CH- and -C(C₁-C₆ alkyl)=CH-; or R¹² together with an adjacent R¹³, or R¹² together with an adjacent R¹⁴, form a - CH=CH- or a -CH=C(C₁-C₆ alkyl)- group;
a and b are independently 0, 1, 2 or 3, provided both are not zero; provided that when R¹³ is -CH=CH- or -C(C₁-C₆ alkyl)=CH-, a is 1; provided that when R¹⁴ is -CH=CH- or -C(C₁-C₆ alkyl)=CH-, b is 1; provided that when a is 2 or 3, the R¹³'s can be the same or different; and provided that when b is 2 or 3, the R¹⁴'s can be the same or different;
R¹⁰ and R¹¹ are independently selected from the group consisting of 1-3 substituents independently selected from the group consisting of (C₁-C₆)alkyl, -OR¹⁹, -O(CO)R¹⁹, -O(CO)OR²¹, -O(CH₂)₁₋₅OR¹⁹, -O(CO)NR¹⁹R²⁰, -NR¹⁹R²⁰, -NR¹⁹(CO)R²⁰, -NR¹⁹(CO)OR²¹,-NR¹⁹(CO)NR²⁰R²⁵, -NR¹⁹SO₂R²¹, -COOR¹⁹, -CONR¹⁹R²⁰, -COR¹⁹,-SO₂NR¹⁹R²⁰, S(O)₀₋₂R²¹, -O(CH₂)₁₋₁₀-COOR¹⁹, -O(CH₂)₁₋₁₀CONR¹⁹R²⁰, -(C₁-C₆ alkylene)-COOR¹⁹,-CH=CH-COOR¹⁹, -CF₃, -CN, -NO₂ and halogen;
Ar¹ can also be pyridyl, isoxazolyl, furanyl, pyrrolyl, thienyl, imidazolyl, pyrazolyl, thiazolyl, pyrazinyl, pyrimidinyl or pyridazinyl;
R¹⁹ and R²⁰ are independently selected from the group consisting of H, (C₁-C₆)alkyl, aryl and aryl-substituted (C₁-C₆)alkyl;
R²¹ is (C₁-C₆)alkyl, aryl or R²⁴-substituted aryl;
R²² is H, (C₁-C₆)alkyl, aryl (C₁-C₆)alkyl, -C(O)R¹⁹ or -COOR¹⁹;
R²³ and R²⁴ are independently 1-3 groups independently selected from the group consisting of H, (C₁-C₆)alkyl, (C₁-C₆)alkoxy, -COOH, NO₂, -NR¹⁹R²⁰, -OH and halogeno; and
R²⁵ is H, -OH or (C₁-C₆)alkoxy.

Ar² is preferably phenyl or R¹¹-phenyl, especially (4-R¹¹)-substituted phenyl. Preferred definitions of R¹¹ are lower alkoxy, especially methoxy, and halogeno, especially fluoro.

Ar¹ is preferably phenyl or R¹⁰-substituted phenyl, especially (4-R¹⁰)-substituted phenyl. A preferred definition of R¹⁰ is halogeno, especially fluoro.

Preferably Q is a lower alkyl or a spiro group as defined above, wherein preferably R¹³ and R¹⁴ are each ethylene and R¹² is 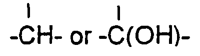 .

A preferred compound of formula I, therefore, is one wherein R¹ is as defined above and in which the remaining variables have the following definitions:
Ar¹ is phenyl or R¹⁰-substituted phenyl, wherein R¹⁰ is halogeno;
Ar² is phenyl or R¹¹ -phenyl, wherein R¹¹ is 1 to 3 substituents independently selected from the group consisting of C₁-C₆ alkoxy and halogeno;
Q is a lower alkyl (i.e C-1 to C-2) with Q = C-2 being preferred, or Q, with the 3-position ring carbon of the azetidinone, forms the group 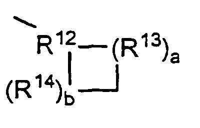
wherein preferably R¹³ and R¹⁴ are each ethylene and a and b are each 1, and wherein R¹² is

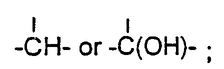

Preferred variables for R¹ groups of the formula 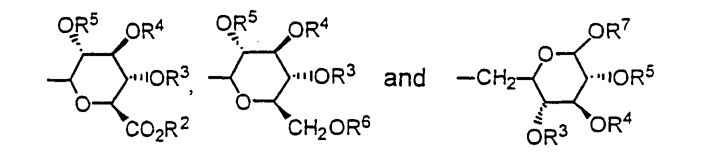 are as follows:
R², R³, R⁴, R⁵, R⁶ and R⁷ are independently selected from the group consisting of H, (C₁-C₆)alkyl, benzyl and acetyl.

Preferred variables for group R¹ of the formula 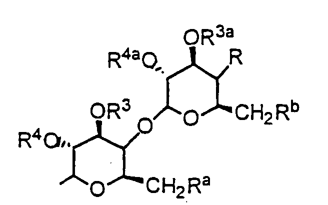 are as follows:
R³, R^{3a}, R⁴ and R^{4a} are selected from the group consisting of H, (C₁-C₆)alkyl, benzyl and acetyl;
R, R^{a} and R^{b} are independently selected from the group consisting of H, - OH, halogeno, -NH₂, azido, (C₁-C₆)alkoxy(C₁-C₆)alkoxy and -W-R³⁰, wherein W is -O-C(O)- or -O-C(O)-NR³¹-, R³¹ is H and R³⁰ is (C₁-C₆)alkyl, -C(O)-(C₁-C₄)alkoxy-(C₁-C₆)alkyl, T , T-(C₁-C₆)alkyl, or T or T-(C₁-C₆)alkyl wherein T is substituted by one or two halogeno or (C₁-C₆)alkyl groups.

Preferred R³⁰ substituents are 2-fluorophenyl, 2,4-difluoro-phenyl, 2,6-dichlorophenyl, 2-methylphenyl, 2-thienylmethyl, 2-methoxy-carbonylethyl, thiazol-2-yl-methyl, 2-furyl, 2-methoxycarbonylbutyl and phenyl. Preferred combinations of R, R^{a} and R^{b} are as follows: 1) R, R^{a} and R^{b} are independently -OH or -O-C(O)-NH-R³⁰, especially wherein R^{a} is -OH and R and R^{b} are -O-C(O)-NH-R³⁰ and R³⁰ is selected from the preferred substituents identified above, or wherein R and R^{a} are -OH and R^{b} is-O-C(O)-NH-R³⁰ wherein R³⁰ is 2-fluorophenyl, 2,4-difluoro-phenyl, 2,6-dichlorophenyl; 2) R^{a} is -OH, halogeno, azido or (C₁-C₆)-alkoxy(C₁-C₆)alkoxy, R^{b} is H, halogeno, azido or (C₁-C₆)alkoxy(C₁-C₆)-alkoxy, and R is -O-C(O)-NH-R³⁰, especially compounds wherein R^{a} is -OH, R^{b} is H and R³⁰ is 2-fluorophenyl; 3) R, R^{a} and R^{b} are independently -OH or -O-C(O)-R³⁰ and R³⁰ is (C₁-C₆)alkyl, T , or T substituted by one or two halogeno or (C₁-C₆)alkyl groups, especially compounds wherein R is -OH and R^{a} and R^{b} are -O-C(O)-R³⁰ wherein R³⁰ is 2-furyl; and 4) R, R^{a} and R^{b} are independently -OH or halogeno. Three additional classes of preferred are compounds are those wherein the C^{1'} anomeric oxy is beta, wherein the C^{2'} anomeric oxy is beta, and wherein the R group is alpha.

R¹ is preferably selected from: 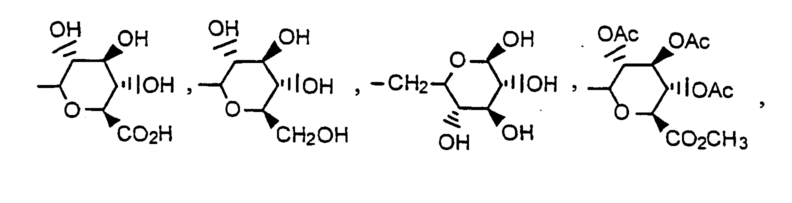 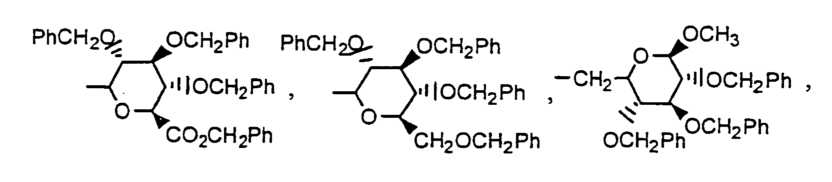 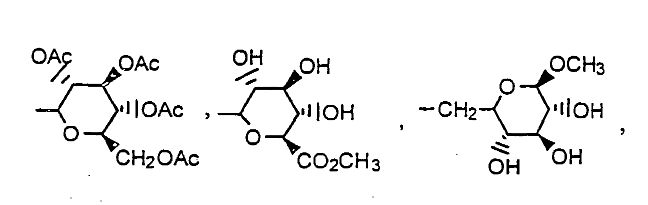 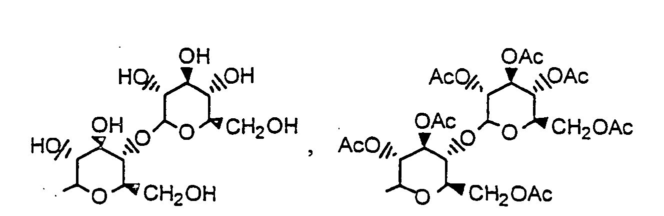 and 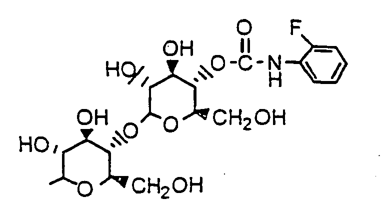 wherein Ac is acetyl and Ph is phenyl.

Thus a preferred compound of this invention is one represented by the formula II: 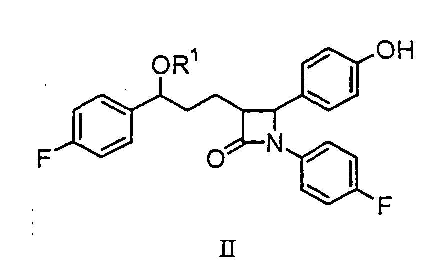 wherein R¹ is defined as above.

A more preferred compound is one represented by formula III:

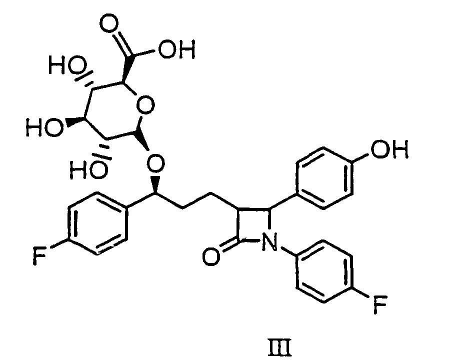

This invention also relates to the method of using a sugar-substituted 2-azetidinone, especially one of furmula I, for treating or preventing atherosclerosis or reducing plasma cholesterol levels comprising administering to a mammal in need of such treating, preventing or reducing an effective amount of a compound of formula I.

In another aspect, the invention relates to a pharmaceutical composition comprising a sugar-substituted 2-azetidinone, especially one of formula I, and a pharmaceutically acceptable carrier.

The present invention also relates to a method of reducing hepatic cholesterol ester levels, a method of reducing plasma cholesterol levels, and to a method of treating or preventing atherosclerosis, comprising administering to a mammal in need of such treatment an effective amount of a combination of a sugar-substituted 2-azetidinone of this invention, especially one of formula I, and a cholesterol biosynthesis inhibitor. That is, the present invention relates to the use of a sugar-substituted 2-azetidinone in combination with a cholesterol biosynthesis inhibitor (and, similarly, use of a cholesterol biosynthesis inhibitor in combination with a sugar-substituted 2-azetidinone) to treat or prevent athersclerosis or to reduce plasma cholesterol levels.

In yet another aspect, the invention relates to a pharmaceutical composition comprising an effective amount of a combination of a sugar-substituted 2-azetidinone and a cholesterol biosynthesis inhibitor and a pharmaceutically acceptable carrier. In a final aspect, the invention relates to a kit comprising in one container an effective amount of a sugar-substituted 2-azetidinone in a pharmaceutically acceptable carrier, and in a separate container, an effective amount of a cholesterol biosynthesis inhibitor in a pharmaceutically acceptable carrier.

### DETAILED DESCRIPTION:

As used herein, the term "alkyl" or "lower alkyl" means straight or branched alkyl chains of 1 to 6 carbon atoms and "alkoxy" similarly refers to alkoxy groups having 1 to 6 carbon atoms.

"Alkenyl" means straight or branched carbon chains having one or more double bonds in the chain, conjugated or unconjugated. Similarly, "alkynyl" means straight or branched carbon chains having one or more triple bonds in the chain. Where an alkyl, alkenyl or alkynyl chain joins two other variables and is therefore bivalent, the terms alkylene, alkenylene and alkynylene are used.

"Cycloalkyl" means a saturated carbon ring of 3 to 6 carbon atoms, while "cycloalkylene" refers to a corresponding bivalent ring, wherein the points of attachment to other groups include all positional isomers.

"Halogeno" refers to fluorine, chlorine, bromine or iodine radicals.

"Aryl" means phenyl, naphthyl, indenyl, tetrahydronaphthyl or indanyl. "Phenylene" means a bivalent phenyl group, including ortho, meta and para-substitution.

"Amino acid" refers to natural and unnatural amino acids and includes but is not limited to Alanine, Arginine, Asparagine, Aspartic Acid, Cysteine, Glycine, Leucine, Serine and Valine.
R²⁴-benzyl and R²⁴-benzyloxy refer to benzyl and benzyloxy radicals which are substituted on the phenyl ring.

The above statements, wherein, for example, R¹⁹, R²⁰ and R²⁵ are said to be independently selected from a group of substituents, means that R¹⁹, R²⁰ and R²⁵ are independently selected, but also that where an R¹⁹, R²⁰ or R²⁵ variable occurs more than once in a molecule, those occurrences are independently selected (e.g., if R¹⁰ is -OR¹⁹ wherein R¹⁹ is hydrogen, R¹¹ can be -OR¹⁹ wherein R¹⁹ is lower alkyl). Those skilled in the art will recognize that the size and nature of the substituent(s) will affect the number of substituents which can be present.

Compounds of the invention have at least one asymmetrical carbon atom and therefore all isomers, including diastereomers and rotational isomers are contemplated as being part of this invention. The invention includes α and β stereoisomers in optically pure form and in admixture, including racemic mixtures. Isomers can be prepared using conventional techniques, either by reacting optically pure or optically enriched starting materials or by separating isomers of a compound of formula I.

Compounds of the invention with an amino group can form pharmaceutically acceptable salts with organic and inorganic acids. Examples of suitable organic and inorganic acids for salt formation are hydrochloric, sulfuric, phosphoric, acetic, citric, oxialic, malonic, salicylic, malic, fumaric, succinic, ascorbic, maleic, methanesulfonic and other organic and inorganic carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

Cholesterol biosynthesis inhibitors for use in combination with compounds of the present invention include HMG CoA reductase inhibitors such as lovastatin, pravastatin, fluvastatin, simvastatin, atorvastatin, NK-104 (itavastatin), and ZD4522; HMG CoA synthetase inhibitors, for example L-659,699 ((E,E-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride). Preferred HMG CoA reductase inhibitors are lovastatin, pravastatin, fluvastatin, atorvastatin and simvastatin. The HMG CoA reductase inhibitor, simvastatin is most preferred.

The cholesterol-lowering 2-azetidinone portions of the compounds of formula I can be prepared by known methods.

Sugars and the derivatives thereof as defined by R¹ the substituents defined above, are known in the art or are readily prepared by known methods.

Preferably, the reactions described above involve a sugar derivative wherein the non-reactive hydroxy groups are protected by suitable protecting groups as defined above for R², R³, R^{3a}, R⁴, R^{4a}, R⁵ and R⁷ other than hydrogen, preferably lower alkyl, acetyl or benzyl, which groups can be removed after the reaction to provide the sugar conjugate. When the 1- and 4-position side chains of the 2-azetidinone include substituent groups which are reactive under the conditions used, said reactive groups are protected by suitable protecting groups prior to reaction with the sugar or the derivative thereof, and the protecting groups are subsequently removed. Depending on the nature of the protecting groups, the protecting groups on the sugar portion and on the 1- and 4-position side chains of the azetidinone can be removed sequentially or simultaneously.

Reactive groups not involved in the above processes can be protected during the reactions with conventional protecting groups which can be removed by standard procedures after the reaction. The following Table 1 shows some typical protecting groups:

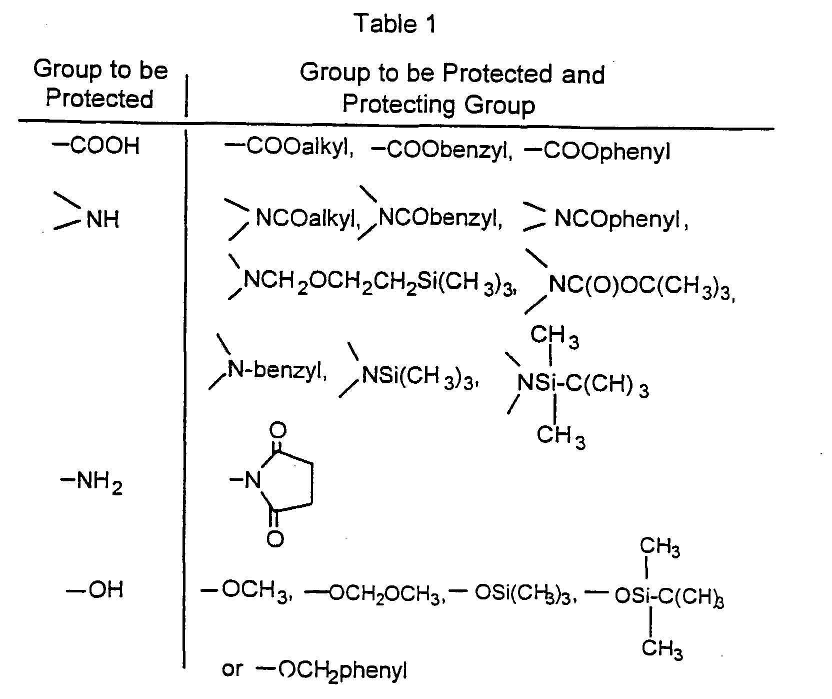

Compared to the 2-azetidinone cholesterol lowering agents which are not sugar substituted, the compounds of this invention have several pharmacological and physical advantages. The compounds are absorbed at a slower rate, give lower plasma levels and higher intestinal levels. Previous testing indicated the intestine as the likely site of activity of the 2-azetidinone compounds lacking a sugar substituent. See van Heek, M. et al, "In vivo mechanism-based discovery of a potent cholesterol absorption inhibitor (SCH 58235) through the identification of the active metabolites of SCH 48461," J. Pharmacol Exp. Ther., 283 (1997), pp. 157-163, and van Heek M. et al, "Comparison of the activity and deposition of the novel cholesterol absorption inhibitor, SCH 58235, and its glucuronide," Br. J. Pharmacol., 129, (2001) pp. 1748-1754. The instantly claimed compounds, which are excreted in the bile, provide efficient delivery of the compound to the desired site while minimizing systemic exposure, thereby decreasing potential toxicity problems.

In addition to the compound aspect, the present invention also relates to a method of lowering plasma cholesterol levels, which method comprises administering to a mammal in need of such treatment a hypocholesterolemic effective amount of a compound of formula I of this invention. The compound is preferably administered in a pharmaceutically acceptable carrier suitable for oral administration.

The present invention also relates to a pharmaceutical composition comprising a compound of formula I of this invention and a pharmaceutically acceptable carrier. The compounds of formula I can be administered in any conventional oral dosage form such as capsules, tablets, powders, cachets, suspensions or solutions. The formulations and pharmaceutical compositions can be prepared using conventional pharmaceutically acceptable excipients and additives and conventional techniques. Such pharmaceutically acceptable excipients and additives include non-toxic compatible fillers, binders, disintegrants, buffers, preservatives, anti-oxidants, lubricants, flavorings, thickeners, coloring agents, emulsifiers and the like.

The effective amount of a compound of formula I is in the range of about 0.001 to about 30 mg/kg of body weight per day, preferably about 0.001 to about 1 mg/kg in single or divided doses. For an average body weight of 70 kg, the effective amount is therefore from about 0.1 to about 100 mg of drug per day, given in a single dose or 2-4 divided doses. The exact dose, however, is determined by the attending clinician and is dependent on the potency of the compound administered, the age, weight, condition and response of the patient.

For the combinations of this invention wherein the substituted azetidinone is administered in combination with a cholesterol biosynthesis inhibitor, the typical daily dose of the cholesterol biosynthesis inhibitor is 0.1 to 80 mg/kg of mammalian weight per day administered in single or divided dosages, usually once or twice a day: for example, for HMG CoA reductase inhibitors, about 10 to about 40 mg per dose is given 1 to 2 times a day, giving a total daily dose of about 10 to 80 mg per day, and for the other cholesterol biosynthesis inhibitors, about 1 to 1000 mg per dose is given 1 to 2 times a day, giving a total daily dose of about 1 mg to about 2 g per day. The exact dose of any component of the combination to be administered is determined by the attending clinician and is dependent on the potency of the compound administered, the age, weight, condition and response of the patient.

Where the components of a combination are administered separately, the number of doses of each component given per day may not necessarily be the same, e.g. where one component may have a greater duration of activity, and will therefore need to be administered less frequently.

Since the present invention relates to the reduction of plasma cholesterol levels by treatment with a combination of active ingredients wherein said active ingredients may be administered separately, the invention also relates to combining separate pharmaceutical compositions in kit form. That is, a kit is contemplated wherein two separate units are combined: a cholesterol biosynthesis inhibitor pharmaceutical composition and a sugar-substituted 2-azetidinone absorption inhibitor pharmaceutical composition. The kit will preferably include directions for the administration of the separate components. The kit form is particularly advantageous when the separate components must be administered in different dosage forms (e.g. oral and parenteral) or are administered at different dosage intervals.

We have found that the compounds of this invention lower plasma lipid levels and hepatic cholesterol ester levels. Compounds of this invention have been found to inhibit the intestinal absorption of cholesterol and to significantly reduce the formation of liver cholesteryl esters in animal models. Thus, compounds of this invention are hypocholesterolemic agents by virtue of their ability to inhibit the esterification and/or intestinal absorption of cholesterol; they are therefore useful in the treatment and prevention of atherosclerosis in mammals, in particular in humans.

Compounds 6A and Example 1 below disclosed in US Patent Nos. 5,767,115 and 5,756,470 respectively, demonstrate pharmacological activity as hypocholesterolemic agents.

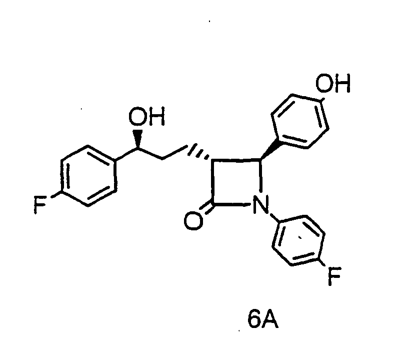

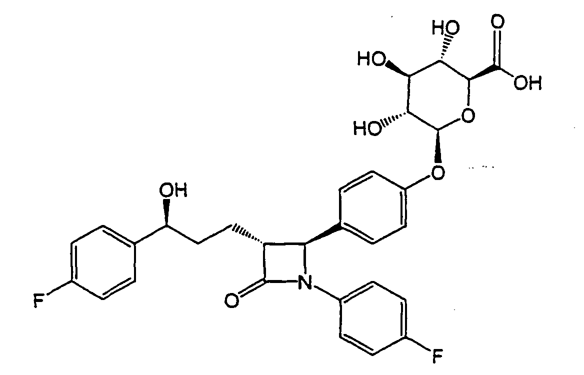

### Example 1

The in vivo activity (see Table 1 below) of the compounds 6A and Example 1 above, can be determined by the following procedure.

### In Vivo Assay of Hypolipidemic Agents Using the Hyperlipidemic Hamster

Hamsters are separated into groups of six and given a controlled cholesterol diet (Purina Chow #5001 containing 0.5% cholesterol) for seven days. Diet consumption is monitored to determine dietary cholesterol exposure in the presence of test compounds. The animals are dosed with the test compound once daily beginning with the initiation of diet. Dosing is by oral gavage of 0.2mL of corn oil alone (control group) or solution (or suspension) of test compound in corn oil. All animals moribund or in poor physical condition are euthanized. After seven days, the animals are anesthetized by IM injection of ketamine and sacrificed by decapitation. Blood is collected into Vacutainer™ tubes containing EDTA for plasma total cholesterol and triglyceride analysis and the liver excised for free and esterified cholesterol and triglyceride tissue analysis. Data is reported as percent reduction of plasma cholesterol and hepatic cholesterol esters versus control levels.

Data is reported as percent change (i.e., percent reduction in plasma cholesterol and in hepatic cholesterol esters) versus control, therefore, negative numbers indicate a positive cholesterol-lowering effect. The assay results are shown in Table 1 below.

**TABLE 1**

| | % Reduction in Plasma Cholesterol | % Reduction in Cholesterol Esters | Dose mg/kg |
|---|---|---|---|
| Example 1 | -58 | -95 | 3 |
| 6A | -59 | -95 | 1 |

Experiment 3 described below demonstrates that both the compound of formula III and Example 1 yield Compound 6A (all shown herein above) following hydrolysis with β-glucuronidase. Experiment Nos. 1 and 2 confirm that Compound 6A yields both Example 1 and the compound of formula III following incubations of Compound 6A with GI tract microsomes or UGT2B7. Since both Compound 6A and Example 1 are shown to demonstrate pharmacological activity (Table 1), the compounds of formulas I, II and III of the present invention are expected to exert similar pharmacological activity.

### Experimental

1. Incubations of Compound 6A with pooled human liver microsomes (n=10) supplemented with Uridine 5'-diphosphate-glucuronic acid (UDPGA) yielded one Compound 6A-glucuronide (retention time ∼7 min) consistent with Example 1 (phenolic glucuronide). However, incubations of Compound 6A with pooled (n=4) and two individuals human jejunum microsomes supplemented with UDPGA yielded two distinct Compound 6A-glucuronides (retention times ∼7 and ∼9 min) consistent with Example 1 (phenolic) and Compound III (benzylic) glucuronides, respectively. LC/MS analysis showed that both peaks have m/z 584.
2. Compound 6A was incubated with commercially available 9 recombinant cDNA expressed human UDP-Glucuronosyltransferases (UGT supersomes) in the presence of UDPGA **(TABLE 2).** Supersomes UGT1A1 and UGT1A3 yielded exclusively Example 1. Incubations with UGT2B7 supersomes yielded mainly Compound III accompanied by a small amount of Example 1.

**TABLE 2**

| Screening of UGT isozymes and Formation of Compound 6A-Glucuronides with 100 µM Compound 6A | | |
|---|---|---|
| Human UGT supersomes + UDPGA | % Conversion to Example 1 | % Conversion to Compound III |
| UGT1A1 | 79.50 | 0 |
| UGT1A3 | 73.40 | 0 |
| UGT1A4 | 0 | 0.78 |
| UGT1A6 | 0 | 0 |
| UGT1A7 | 0 | 0 |
| UGT1A9 | 0.30 | 0.50 |
| UGT1A10 | 0 | 0 |
| **UGT2B7** | 0.50 | 6.16 |
| UGT2B15 | 6.06 | 0 |
| Insect control | 0 | 0 |

3. β-Glucuronidase hydrolysis of the mixture of Example 1 and Compound III (Compound 6A-benzylic glucuronides) obtained from jejunum microsomes (5, 10, 20, 30 and 180 min, **TABLE 3**) demonstrates that Example 1 was hydrolyzed at a faster rate than Compound III. After hydrolyzing for 18h, both peaks were hydrolyzed to form a single Compound 6A peak.

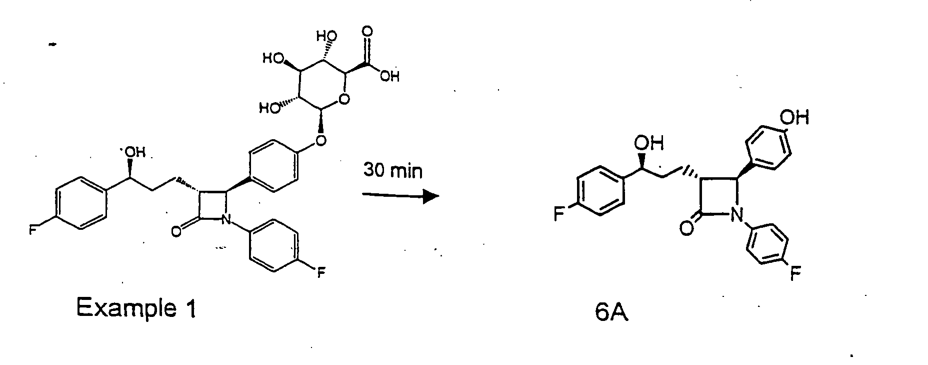

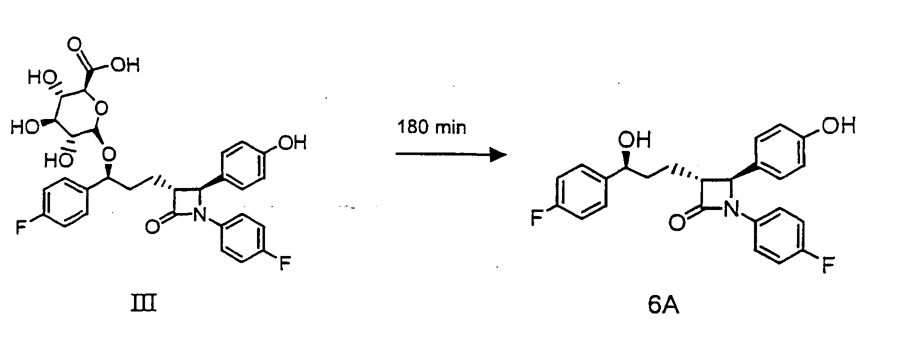

**TABLE 3**

| Hydrolysis with β-Glucuronidase after 2 hr Incubation of Human Jejunum Microsomes with 50 µM Compound 6A Supplemented with UDPGA | | | |
|---|---|---|---|
| Hydrolysis time | % of Example 1 (Phenolic Glucuronide) | % of Compound III (Benzylic Glucuronide) | % of Compound 6A |
| No hydrolysis | 31.68 | 32.14 | 32.06 |
| 5 min | 2.23 | 19.30 | 68.10 |
| 10 min | 1.04 | 18.58 | 61.88 |
| 20 min | 0.77 | 15.12 | 66.02 |
| 30 min | 0 | 11.22 | 80.14 |
| 180 min | 0 | 6.5 | 84.67 |
| Control: 180 min, No microsomes, No UDPGA | - | - | 72.92 |

### Scale Up Production and Structure Identification of Compound III

### Scale-up Preparation and Extraction of Compound III

Scale up production of Compound III was performed using 1.23 mg (0.05 mM) of ¹⁴C-SCH 58235 and 60 mg protein of cDNA expressed recombinant human UGT2B7 supersomes supplemented with UDPGA (2 mM) in 60 ml Tris buffer, pH 7.4. The incubation was carried out for 2 hr at 37°C and subjected to solid phase extraction (SPE). The methanol elution from SPE was dried and Compound III was further purified as described below.

### Isolation of Compound III for LC/NMR Analysis

Compound III was isolated using preparative HPLC with fraction collection. The dried residue from SPE methanol elution was reconstituted in ca. 3 mL of CH₃OH and centrifuged (16,000 *g*) to remove solid precipitate. Methanol was evaporated and the residue redissolved in ca. 2 mL of CH₃OH:DMSO (20:80, v:v). The preparative HPLC column (Inertsil C8, 250 x 20 mm) provided a retention time of ca. 15.0 and 20.6 min for Example 1 and Compound III, respectively. Compound III was isolated using 200 µL injections (10 in total) onto the preparative column collecting 0.5 min fractions. Compound III eluted in fractions numbered 37 (18.5 min) through 44 (22.0 min) for each injection. These fractions were within the observed retention time for the Compound III were analyzed by LC-MS/MS. The fractions (18.5 - 22 min) were combined and dried.

### Determination of Structure of Compound III by LC/NMR

LC-NMR was carried out using mobile phases of 20 mM ammonium acetate-*d*_{*3*} (pH 7.0) and acetonitrile. The HPLC gradient was 30% acetonitrile for 10 minutes, and then went up to 40% for 20 minutes. The metabolite eluted at approximately 10 minute. LC-NMR was conducted in stop-flow mode on the metabolite peak apex. 1 D proton and 2D proton-proton correlation spectra were recorded on Varian 600 MHz NMR spectrometer at 20°C. Corresponding NMR data were obtained on synthetic standards Compound 6A and Example 1 (Compound 6A-phenolic glucuronide). Based on the NMR data of the sample and the comparison with those from the standards, the proton assignments for this metabolite (MW 585) were made. The structure of this metabolite was identified to be Compound 6A-benzylic-glucuronide (Compound III).

## Claims

1. A compound represented by the structural formula I 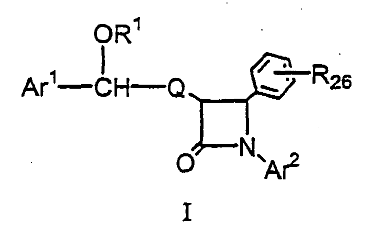 or a pharmaceutically acceptable salt thereof, wherein
R²⁶ is selected from the group consisting of:
a) OCH₃;
b)fluorine and
c) chlorine.
R¹ is selected from the group consisting of H, 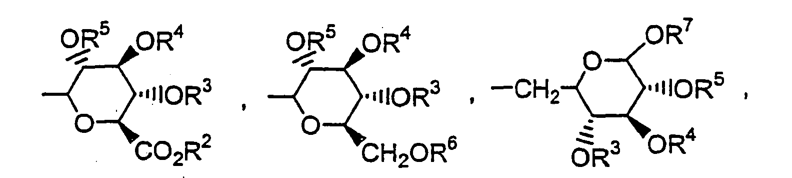 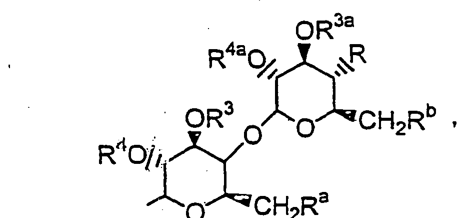 -SO₃H ;natural and unnatural amino acids.
R, R^{a} and R^{b} are independently selected from the group consisting of H, -OH, halogeno, -NH₂, azido, (C₁-C₆)alhoxy(C₁-C₆)-alkoxy or -W-R³⁰; W is independently selected from the group consisting of -NH-C(O)-, -O-C(O)-, -O-C(O)-N(R³¹)-, -NH-C(O)-N(R³¹)- and -O-C(S)-N(R³¹)-;
R² and R⁶ are independently selected from the group consisting of H, (C₁-C₆)alkyl, aryl and aryl(C₁-C₆)alkyl;
R³, R⁴, R⁵, R⁷, R^{3a} and R^{4a} are independently selected from the group consisting of H, (C₁-C₆)alkyl, aryl(C₁-C₆)alkyl, -C(O)(C₁-C₆)alkyl and -C(O)aryl;
R³⁰ is independently selected form the group consisting of R³²-substituted T, R³²-substituted-T-(C₁-C₆)alkyl, R³²-substituted-(C₂-C₄)alkenyl, R³²-substituted-(C₁-C₆)alkyl, R³²-substituted-(C₃-C7)cycloalkyl and R³²-substituted-(C₃-C7)cycloalkyl(C₁-C₆)alkyl;
R³¹ is independently selected from the group consisting of H and (C₁-C₄)alkyl;
T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
R³² is independently selected from 1-3 substituents independently selected from the group consisting of H, halogeno, (C₁-C₄)alkyl, -OH, phenoxy, -CF₃, -NO₂, (C₁-C₄)alkoxy, methylenedioxy, oxo, (C₁-C₄)alkylsulfanyl, (C₁-C₄)alkylsulfinyl, (C₁-C₄)alkylsulfonyl, -N(CH₃)₂, -C(O)-NH(C₁-C₄)alkyl, -C(O)-N((C₁-C₄)alkyl)₂, -C(O)-(C₁-C₄)alkyl, -C(O)-(C₁-C₄)alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a (C₁-C₄)alkoxycarbonyl-substituted pyrrolidinyl, piperidinyl, N-methylpiperazinyl, indolinyl or morpholinyl group;
Ar¹ is aryl or R¹⁰-substituted aryl;
Ar² is aryl or R¹¹-substituted aryl;
Q is -(CH₂)_{q}-, wherein q is 2-6, or, with the 3-position ring carbon of the azetidinone, forms the spiro group 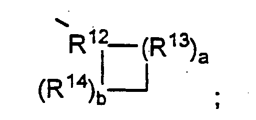
R¹² is 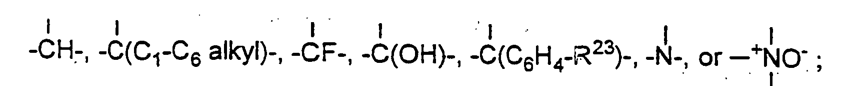
R¹³ and R¹⁴ are independently selected from the group consisting of -CH₂-,-CH(C₁-C₆ alkyl)-, -C(di-(C₁-C₆) alkyl), -CH=CH- and -C(C₁-C₆ alkyl)=CH-; or R¹² together with an adjacent R¹³, or R¹² together with an adjacent R¹⁴, form a-CH=CH- or a -CH=C(C₁-C₆ alkyl)- group;
a and b are independently 0, 1, 2 or 3, provided both are not zero; provided that when R¹³ is -CH=CH- or -C(C₁-C₆ alkyl)=CH-, a is 1; provided that when R¹⁴ is - CH=CH- or -C(C₁-C₆ alkyl)=CH-, b is 1; provided that when a is 2 or 3, the R¹³'s can be the same or different; and provided that when b is 2 or 3, the R¹⁴'s can be the same or different;
R¹⁰ and R¹¹ are independently selected from the group consisting of 1-3 substituents independently selected from the group consisting of (C₁-C₆)alkyl, -OR¹⁹, -O(CO)R¹⁹, -O(CO)OR²¹, -O(CH₂)₁₋₅OR¹⁹, -O(CO)NR¹⁹R²⁰, -NR¹⁹R²⁰, -NR¹⁹(CO)R²⁰, -NR¹⁹(CO)OR²¹,-NR¹⁹(CO)NR²⁰R²⁵, -NR¹⁹SO₂R²¹, -COOR¹⁹, -CONR¹⁹R²⁰, -COR¹⁹,-SO₂NR¹⁹R²⁰, S(O)₀₋₂R²¹, -O(CH₂)₁₋₁₀-COOR¹⁹, -O(CH₂)₁₋₁₀CONR¹⁹R²⁰, -(C₁-C₆ alkylene)-COOR¹⁹,-CH=CH-COOR¹⁹, -CF₃, -CN, -NO₂ and halogen;
Ar¹ can also be pyridyl, isoxazolyl, furanyl, pyrrolyl, thienyl, imidazolyl, pyrazolyl, thiazolyl, pyrazinyl, pyrimidinyl or pyridazinyl;
R¹⁹ and R²⁰ are independently selected from the group consisting of H, (C₁-C₆)alkyl, aryl and aryl-substituted (C₁-C₆)alkyl;
R²¹ is (C₁-C₆)alkyl, aryl or R²⁴-substituted aryl;
R²² is H, (C₁-C₆)alkyl, aryl (C₁-C₆)alkyl, -C(O)R¹⁹ or -COOR¹⁹;
R²³ and R²⁴ are independently 1-3 groups independently selected from the group consisting of H, (C₁-C₆)alkyl, (C₁-C₆)alkoxy, -COOH, NO₂, -NR¹⁹R²⁰, -OH and halogeno; and
R²⁵ is H, -OH or (C₁-C₆)alkoxy.

2. A compound of claim 1 wherein Ar¹ is phenyl or R¹⁰-substituted phenyl and Ar² is phenyl or R¹¹ -phenyl.

3. A compound of claim 2 wherein R¹⁰ is halogeno and R¹¹ is lower alkoxy or halogeno.

4. A compound of claim 1 wherein R¹ is selected from the group consisting of: 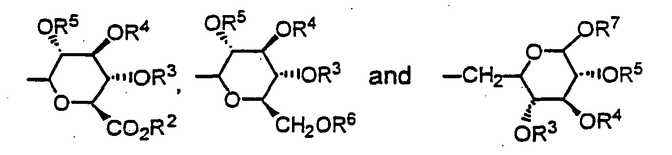 wherein:
R², R³, R⁴, R⁵, R⁶ and R⁷ are independently selected from the group consisting of H, (C₁-C₆)alkyl, benzyl and acetyl.

5. A compound of claim 1 wherein R¹ is selected from: 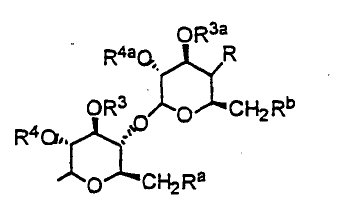 wherein:
R³, R^{3a}, R⁴ and R^{4a} are selected from the group consisting of H, (C₁-C₆)alkyl, benzyl and acetyl;
R, R^{a} and R^{b} are independently selected from the group consisting of H, -OH, halogeno, -NH₂, azido, (C₁-C₆)alkoxy(C₁-C₆)alkoxy and -W-R³⁰, wherein W is -O-C(O)- or -O-C(O)-NR³¹-, R³¹ is H and R³⁰ is (C₁-C₆)alkyl, -C(O)-(C₁-C₄)alkoxy-(C₁-C₆)alkyl, T, T-(C₁-C₆)alkyl, or T or T-(C₁-C₆)alkyl wherein T is substituted by one or two halogeno or (C₁-C₆)alkyl groups.

6. A compound of claim 5 wherein R³⁰ is 2-fluorophenyl, 2,4-difluorophenyl, 2-methylphenyl, 2-thienylmethyl, 2-methoxycarbonyl-ethyl, thiazol-2-yl-methyl, 2-methoxycarbonylbutyl or phenyl, or W is -O-C(O)- and R³⁰ is (C₁-C₆)alkyl, T , or T substituted by one or two halogeno or (C₁-C₆)alkyl groups.

7. A compound of claim 1 wherein:
Ar¹ is phenyl or R¹⁰-substituted phenyl;
Ar² is phenyl or R¹¹-phenyl:
R¹⁰ is halogeno;
R¹¹ is lower alkoxy or halogeno;
Q is -(CH₂)_{q}-, wherein q is 2-6; or Q, with the 3-position ring carbon of the azetidinone, forms the group 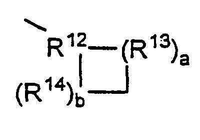 wherein R¹³ and R¹⁴ are each ethylene and a and b are each 1, and wherein R¹² is 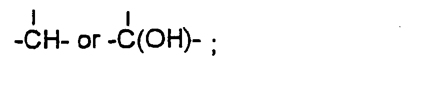
R¹ is selected from the group consisting of 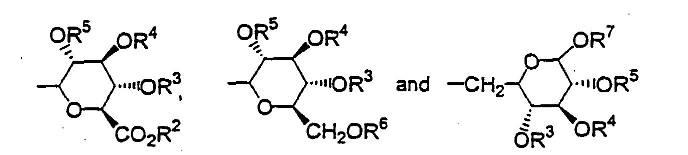
wherein R², R³, R⁴, R⁵, R⁶ and R⁷ are independently selected from the group consisting of H, (C₁-C₆)alkyl, benzyl and acetyl; or R¹ is 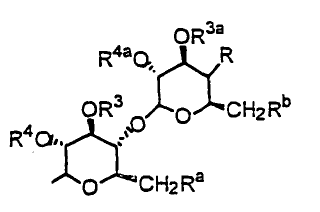 wherein R³, R^{3a}, R⁴ and R^{4a} are selected from the group consisting of H, (C₁-C₆)alkyl, benzyl and acetyl; and R, R^{a} and R^{b} are independently selected from the group consisting of H, -OH, halogeno, -NH₂, azido, (C₁-C₆)alkoxy(C₁-C₆)alkoxy and -W-R³⁰, wherein W is -O-C(O)- or -O-C(O)-NR³¹-, R³¹ is H and R³⁰ is (C₁-C₆)alkyl, -C(O)-(C₁-C₄)alkoxy-(C₁-C₆)alkyl, T , T-(C₁-C₆)alkyl, or T or T-(C₁-C₆)alkyl wherein T is substituted by one or two halogeno or (C₁-C₆)alkyl groups.

8. A compound of claim 7 wherein R¹ is selected from the group consisting of 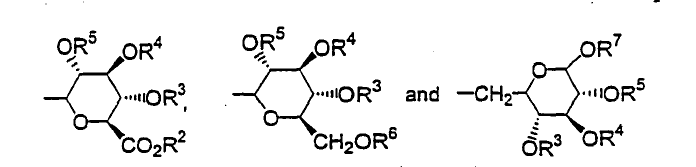 wherein R², R³, R⁴, R⁵, R⁶ and R⁷ are independently selected from the group consisting of H, (C₁-C₆)alkyl, benzyl and acetyl.

9. A compound of the formula II: 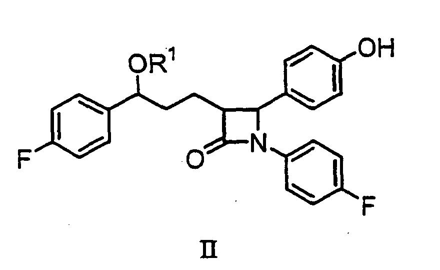 wherein R¹ is selected from the group consisting of H, 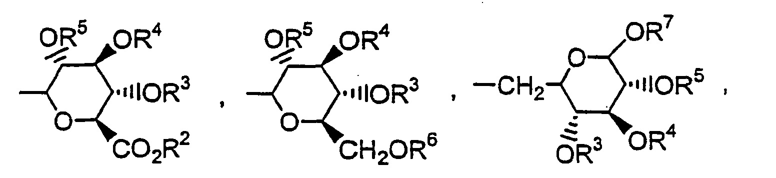 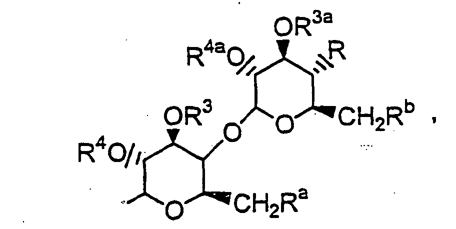 -SO₃H; natural and unnatural amino acids;
and wherein R², R³, R^{3a}, R⁴, R^{4a}, R⁵, R⁶ and R⁷ are independently selected from the group consisting of H, (C₁-C₆)alkyl, benzyl and acetyl;
R, R^{a} and R^{b} are independently selected from the group consisting of H, -OH, halogeno, -NH₂, azido, (C₁-C₆)alkoxy(C₁-C₆)alkoxy and -W-R³⁰, wherein W is -O-C(O)- or -O-C(O)-NR³¹-, R³¹ is H and R³⁰ is (C₁-C₆)alkyl, -C(O)-(C₁-C₄)alkoxy-(C₁-C₆)alkyl, T , T-(C₁₋C₆)alkyl, or T or T-(C₁-C₆)alkyl wherein T is substituted by one or two halogeno or (C₁-C₆)alkyl groups.

10. A compound of the formula III:

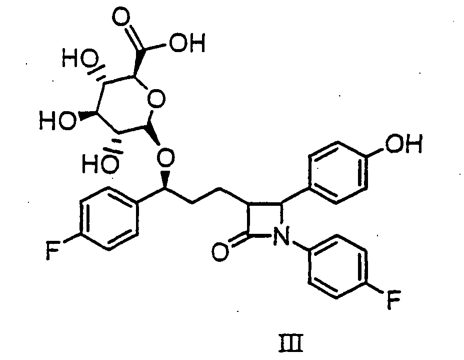

11. The use of an effective amount of a compound of claim 1 or 10 for the preparation of a medicament for lowering cholesterol levels in a mammal in need of such treatment.

12. A pharmaceutical composition comprising an effective amount of a compound of claim 1 or 10 in a pharmaceutically acceptable carrier.

13. A pharmaceutical composition for the treatment or prevention of atherosclerosis, or for the reduction of cholesterol levels, comprising an effective amount of a combination of a compound as defined in claim 1 or 10, a cholesterol biosynthesis inhibitor and a pharmaceutically acceptable carrier

14. The pharmaceutical composition of claim 13 wherein the cholesterol biosynthesis inhibitor is selected from the group consisting of lovastatin, pravastatin, fluvastatin, simvastatin, atorvastatin, L-659,699, squalestatin 1, NB-598, NK-104 (itavastatin) and ZD4522.

15. The pharmaceutical composition of claim 13 wherein the cholesterol biosynthesis inhibitor in simvastatin.

16. A kit comprising in separate containers in a single package pharmaceutical compositions for use in combination to treat or prevent atherosclerosis or to reduce cholesterol levels which comprises in one container an effective amount of a cholesterol biosynthesis inhibitor in a pharmaceutically acceptable carrier, and in a second container, an effective amount of a compound of claim 1 or 10 in a pharmaceutically acceptable carrier.

17. The use of an effective amount of a combination of a cholesterol biosynthesis inhibitor and a compound of claim 1 or 10 for the preparation of a medicament for treating or preventing atherosclerosis or reducing cholesterol levels for simultaneous or sequential administration.

18. The use of claim 17, wherein the cholesterol biosynthesis inhibitor is selected from the group consisting of lovastatin, pravastatin, fluvastatin, simvastatin, atorvastatin, L-659,699, squalestatin 1, NB-598, NK-104 (itavastatin) and ZD4522.

19. The use of claim 17 wherein the cholesterol biosynthesis inhibitor is simvastatin.
